# EUROPÄISCHE PATENTSCHRIFT

(11) **EP 0 532 511 B1**
(45) Veröffentlichungstag und Bekanntmachung des Hinweises auf die Patenterteilung: **02.11.1994**
(21) Anmeldenummer: 91907466.6
(22) Anmeldetag: 10.04.1991
(51) Int. Cl.: C07C 69/608, C09K 19/30, C09K 19/34, C09K 19/12, C07C 69/76, C07C 69/616, C07C 69/753, C07C 255/57

(54) **TETRASUBSTITUIERTE METHANE MIT FLÜSSIGKRISTALLINEN EIGENSCHAFTEN**
TRETRA-SUBSTITUTED METHANES WITH LIQUID CRYSTAL PROPERTIES
DERIVES DU METHANE TETRA-SUBSTITUES DOTES DE PROPRIETES DE CRISTAUX LIQUIDES

(30) Priorität: 12.04.1990 DE 4011812
(43) Veröffentlichungstag der Anmeldung: 24.03.1993
(73) Patentinhaber: Consortium für elektrochemische Industrie GmbH, D-81379 München (DE)
(72) Erfinder: EIDENSCHINK, Rudolf, D-6501 Bodenheim (DE)
(86) Internationale Anmeldenummer: EP9100681
(87) Internationale Veröffentlichungsnummer: WO9116295

(56) Entgegenhaltungen:
- EP-A- 0 163 495
- LIQUID CRYSTALS, vol. 8, no. 6, 1990; R. EIDENSCHINK: "Liquid-crystalline behaviour of molecules with tetrahedral symmetry", pp. 879-884

## Beschreibung

Gegenstand der Erfindung sind neue tetrasubstituierte Methane der allgemeinen Formel

C [-Sp-M-R]₄ (I)

worin die Reste -Sp-M-R gleich oder verschieden sein können, wobei
Sp Spacerelemente darstellt und jeweils unabhängig voneinander einen unsubstituierten oder einen einfach mit Halogen, -CN oder -CF₃ substituierten Alkylen- oder Alkenylenrest mit 1 bis 12 Kohlenstoffatomen, worin auch eine oder zwei nichtbenachbarte CH₂-Gruppen durch -O-, -CO-, -COO-, -OOC-, -CONH- oder -OCOO- ersetzt sein können, oder eine Einfachbindung, M eine nach stehend definierte mesogene Gruppe und R Wasserstoff, Halogen, -CN, -CF₃, -OCF₃, -NO₂, -N(CH₃)₂, einen unsubstituierten, einen ein- oder mehrfach durch -CN, -NH₂, -CF₃ oder Halogen substituierten, gerad- oder verzweigtkettigen Alkylrest oder Alkenylrest mit jeweils 1 bis 12 Kohlenstoffatomen, in denen eine oder mehrere nichtbenachbarte CH₂-Gruppen durch die Reste -O-, -CO-, -OOC-, -COO- oder -OCOO- ersetzt sein können,
bedeuten.

Die erfindungsgemäßen neuen Methanderivate der allgemeinen Formel I und II (s. weiter unten) sind thermotrope flüssigkristalline Verbindungen; aufgrund ihres hierfür neuartigen molekularen Bauprinzips verbreitern sie allgemein die bekannte Palette der flüssigkristallinen Substanzen, mit denen Gemische mit günstigen anwendungstechnischen Eigenschaften hergestellt werden können.

Die Moleküle von mesogenen Verbindungen, die bislang eine technische Bedeutung in der Displaytechnik und als Temperaturanzeigen erlangt haben, weisen demgegenüber eine langgestreckte Form auf (vgl. K.J. Toyne in Thermotropic Liquid Crystals, John Wiley & Sons, Ed., G.W. Gray, 1987, S.28ff); diese bilden nematische, cholesterische und smektische Phasen. Darüber hinaus werden thermotrop flüssigkristalline Phasen von diskusförmigen Molekülen gebildet (vgl. C. Destrade u.a., Mol. Cryst. Liq. Cryst. Bd.106, S.121 (1984)).

Es wurde nun überraschenderweise gefunden, daß die tetrasubstituierten Methane der allgemeinen Formel

C[Sp-A-(Z₁-A₁)ₘ-(Z₂-A₂)ₙ-R]₄, (II)

worin -A-(Z₁-A₁)ₘ-(Z₂-A₂)ₙ- für M steht und
Z₁, Z₂
jeweils unabhängig voneinander
-CH₂CH₂-, -COO-, -OOC-, -OCH₂-, CH₂O-, -C≡C-, -N=N- oder eine Einfachbindung;
A, A₁, A₂
jeweils unabhängig voneinander einen unsubstituierten oder einen bis vierfach mit Fluor oder Chlor substituierten 1,4-Phenylenrest, worin auch eine oder zwei CH-Gruppen durch N ersetzt sein können, einen insbesondere trans-Konfiguration aufweisenden 1,4-Cyclohexylenrest, worin auch eine oder zwei nichtbenachbarte CH₂-Gruppen durch -O-ersetzt sein können, einen Piperidin-1,4-diylrest oder einen 1,4-Bicyclo[2.2.2]octylenrest; sowie
n und m
jeweils unabhängig voneinander 0 oder 1, mit der Maßgabe, daß in einem der vier Substituenten des Methans m oder n mindestens einmal 1 ist,
bedeuten, und Sp und R die zuvor genannten Bedeutungen besitzen, sich durch hohe Übergangstemperaturen vom flüssigkristallinen Zustand in die isotrope Phase (Klärpunkt) auszeichnen. Infolgedessen eignen sie sich hervorragend zur Verwendung als chemisch stabile Komponenten in flüssigkristallinen Medien, die ihrerseits aufgrund ihrer günstigen optischen und dielektrischen Anisotropien zur Herstellung elektrooptischer Displays geeignet sind, zumal sie chemisch und thermisch stabil sind.

Solche Vorrichtungen beruhen bekanntlich auf der Änderung einer durch Oberflächeneffekte vorgegebenen Vorzugsrichtung der molekularen Ausrichtung durch ein elektrisches Feld. Hierdurch kann die Schwingungsebene polarisierten Lichtes gedreht oder die Absorption gelöster dichroitischer Farbstoffe geändert werden. Darüber hinaus lassen sich flüssigkristalline Medien zur Herstellung von Temperaturanzeigen, die auf der selektiven Reflexion natürlichen Lichtes beruhen (vgl. H. Kelker u.a., Chem.Ing.Tech., Bd.45, S.1005 (1973)), mit vorteilhaften optischen Eigenschaften herstellen. Hierfür eignen sich solche neuen Verbindungen, die in einem oder mehreren Resten R, Sp, Z₁ und Z₂ optisch aktive Kohlenstoffatome enthalten.

Ferner lassen sich mit den erfindungsgemäßen flüssigkristallinen Medien löschbare optische Speicherelemente herstellen. Das Prinzip solcher Vorrichtungen ist beschrieben bei D. Coates, Thermotropic Liquid Crystals, John Wiley & Sons, Ed. G.W. Gray, 1987, S.99ff).

Die Verbindungen der allgemeinen Formel II zeichnen sich ferner durch eine mit ihrer vergleichsweise hohen molekularen Masse verbundene geringe Flüchtigkeit aus. Deshalb können sie auch in nichtlinear-optischen Schaltelementen, insbesondere in Pockels-Zellen und Frequenzverdopplern, verwendet werden.

Durch geeignete Wahl des Substitutionsmusters der Verbindungen können hohe molekulare Hyperpolarisierbarkeiten zweiter und dritter Ordnung erhalten werden. Zur Erreichung einer makroskopischen Hyperpolarisierbarkeit zweiter Ordnung ist bekanntlich eine nichtzentrosymmetrische Anordnung der Moleküle nötig. Die allgemeinen Zusammenhänge von molekularen Eigenschaften und anwendungstechnischen Parametern sind bekannt (vgl. D.J. Williams u.a., "Nonlinear Optical Properties of Organic Molecules and Crystals", Vol.1, Academic Press, New York (1987) und G.G. Roberts, Adv.Phys., Bd.34, S.475 (1985)).

Durch Wahl der Art der Substituenten können smektische und nematische Medien mit günstigen optischen und dielektrischen Eigenschaften oder cholesterische Medien mit geeigneten Ganghöhen ihrer Schraubenstruktur erhalten werden. Sie zeichnen sich darüber hinaus allgemein wegen ihrer vergleichsweise hohen Molmasse durch niedrige Dampfdrücke aus.

Die Verbindungen der allgemeinen Formeln I und II eignen sich weiterhin als anisotrope Matrices für spektroskopische Untersuchungen.

Weitere Gegenstände der vorliegenden Erfindung sind somit die Anwendung der neuen Methanderivate, insbesondere der Verbindungen der allgemeinen Formel II, als Komponenten flüssigkristalliner Medien sowie solche Medien enthaltende elektrooptische Anzeigeelemente, Temperaturanzeigen, optische Speicher und nichtlinear-optische Schalter.

Die Verbindunger der allgemeinen Formeln I und II umfassen Methanderivate mit vier gleichen Substituenten
-Sp-M-R bzw. -[Sp-A-(Z₁-A₁)ₘ-(Z₂-A₂)ₙ-R]
und solche, in denen diese Substituenten verschieden sind. Bevorzugt sind diejenigen Methanderivate, die drei gleiche (und einen weiteren verschiedenartigen) Substituenten haben. Besonders bevorzugt sind solche Verbindungen, die vier gleiche Substituenten aufweisen. Die Verbindungen der Formel II können fünf bis zwölf ringförmige Strukturelemente enthalten.

Die Verbindungen mit gleichen Substituenten des Methans umfassen Verbindungen mit je zwei Ringen im Substituenten gemäß den Teilformeln IIa und IIb:

C(Sp-A-A₁-R)₄ (IIa)

C(Sp-A-Z₁-A₁-R)₄ (IIb)

und mit je drei Ringen gemäß den Teilformeln:

C(Sp-A-A₁-A₂-R)₄ (IIc)

C(Sp-A-Z₁-A₁-A₂-R)₄ (IId)

C(Sp-A-Z-₁-A₁-Z₂-A₂-R)₄ (IIe)

C(Sp-A-A₁-Z₂-A₂-R)₄ (IIf)

Darunter sind diejenigen der Teilformeln IIa, IIb und IIc besonders bevorzugt. Die Bedeutung der Symbole R,A,A₁,A₂,Sp,Z₁,Z₂,m und n entspricht der weiter oben genannten. Die ringförmigen Strukturelemente A, A₁ und A₂ in der Formel II werden im folgenden vereinfacht dargestellt: PH steht für eine 1,4-Phenylengruppe, CY für eine 1,4-Cyclohexylengruppe und PY für eine Pyrimidin-2,5-diylgruppe, wobei diese Strukturelemente auch mit Fluor oder Chlor substituiert sein können.

Die Verbindungen der Teilformel IIa umfassen die bevorzugten Teilformeln IIaa bis IIac

C(Sp-PH-PH-R)₄ (IIaa)

C(Sp-PH-CY-R)₄ (IIab)

C(Sp-CY-CY-R)₄ (IIac)

Die Verbindungen der Formel IIb umfassen die bevorzugten Teilformeln IIba und IIbb

C(Sp-PH-CH₂CH₂-CY-R)₄ (IIba)

C(Sp-PH-COO-PH-R)₄ (IIbb)

Die Verbindungen der Formel IIc umfassen die bevorzugten Teilformeln IIca bis IIcc

C(Sp-PH-PH-CY-R)₄ (IIca)

C(Sp-PH-CY-CY-R)₄ (IIcb)

C(Sp-PH-CY-CY-R)₄ (IIcc)

Sp bedeutet bevorzugt -CH₂O-, -CH₂OOC-,
-CH₂O(CH₂)ᵣ-, -CH₂OOC(CH₂)ₛ-, wobei r
und s jeweils 0 bis 10, bevorzugt 1 bis 6 sind, oder die Einfachbindung. Z₁ und Z₂ bedeuten bevorzugt jeweils die Einfachbindung oder einen der Reste -CH₂CH₂-, -OOC- und -COO-.

In den vorstehenden Teilformeln bedeutet R vorzugsweise einen Alkyl-, Alkoxy- oder Alkoxymethylrest. Bedeutet R einen Alkylrest, so kann er geradkettig oder verzweigt sein. Vorzugsweise ist er unverzweigt und bedeutet Methyl, Ethyl, Propyl, Butyl, Pentyl, Hexyl oder Heptyl.

Die 1,4-Cyclohexylengruppen der Formel II haben vorzugsweise trans-Konfiguration.

Die Verbindungen der Formel II können ein oder mehrere asymmetrische C-Atome enthalten. In diesem Fall werden von den Formeln neben optisch aktiven Enantiomeren auch Enantiomerengemische und Racemate erfaßt. Diejenigen Verbindungen der Formel II, die für Polymerisationsreaktionen geeignete Gruppen R haben, eignen sich zur Herstellung flüssigkristalliner Polymerer.

Die neuen Verbindungen der allgemeinen Formel II werden nach an sich allgemein bekannten Verfahren hergestellt. Diese sind beispielsweise dem Sammelwerk Houben-Weyl, Methoden der organischen Chemie, Georg Thieme Verlag, Stuttgart, zu entnehmen.

Es ist in vielen Fällen vorteilhaft, zunächst Vorstufen der allgemeinen Formel III

X-Sp-A-(Z₁-A₁)ₘ-(Z₂-A₂)ₙ-R (III)

herzustellen, wobei X für eine reaktive Gruppe steht, und diese dann mit einem reaktiven tetrakis-substituierten Methan umzusetzen. Sollen die erfindungsgemäßen Methane der Formel II verschiedene Substituenten haben, wird man zweckmäßigerweise unter Isolierung von Zwischenprodukten die reaktiven Gruppen des Methans nacheinander umwandeln. Ist das eingesetzte reaktive tetrakis-substituierte Methan optisch aktiv, führt dieser Weg zu Verbindungen der Formel II mit chiralem Zentralatom. Setzt man mehrere Vorstufen der Formel III gleichzeitig um, erhält man kompliziert zusammengesetzte Mischungen von Verbindungen der Formel II, die auf Grund ihrer niedrigen Schmelzpunkte anwendungstechnische Vorteile aufweisen können.

Verbindungen der Formel II, die die Estergruppe -OOC- oder -COO- enthalten, können in allgemein bekannter Weise durch Umsetzung von Alkoholen mit Carbonsäurehalogeniden oder Carbonsäureanhydriden hergestellt werden. Die Ethergruppen -CH₂O- oder -OCH₂- werden durch Umsetzung von Alkylhalogeniden oder Alkyltosylaten mit Metallalkoholaten oder Metallphenolaten hergestellt. In einem speziellen Verfahren können Tetraether des Pentaerythrits durch Phasentransferkatalyse aus Penaerythrit und Alkylbromiden nach Nouguier und Mchich (J.Org.Chem., Bd.50, S.3296 (1985)) hergestellt werden.

Die direkte Verknüpfung von Aryloxyresten mit dem zentralen Kohlenstoffatom erfolgt zweckmäßigerweise durch Umsetzung von Kupferphenolaten mit Tetrachlorkohlenstoff (Can.J.Chem., Bd.57, S.890 (1979)). Orthoester des Typs
C(O-Zₒ-A-Z₁-Z₂-A₂-R)₄ (Zₒ = Alkylen oder die Einfachbindung) werden durch Umesterung nach allgemein bekannten Verfahren hergestellt.

Verbindungen der Formeln II und III können in an sich bekannter Weise z.B. durch Reduktion von Synthesevorstufen hergestellt werden, wobei -CO- in -CH₂- oder -HCOH- Gruppen, -CH=CH- in -CH₂CH₂-Gruppen, -C≡C- in -CH₂CH₂ Gruppen und aromatische Ringe in alicyclische oder heteroalicyclische Ringe umgewandelt werden.

So können Ketone und Aldehyde nach Wolff-Kishner in alkalischer Lösung mit Hydrazinhydrat zu den entsprechenden Kohlenwasserstoffen umgewandelt werden. Die Reduktion mit gasförmigem Wasserstoff erfolgt in Anwesenheit eines Katalysators bei Temperaturen zwischen O° und 200°C und Drücken zwischen etwa 1 und 200 bar in Lösungsmitteln, wie Ethanol, Tetrahydrofuran oder Eisessig. Als Katalysatoren eignen sich Platin und Palladium oder auch Raney-Nickel. Die Umwandlung von Carbonylverbindungen in Alkohole erfolgt zweckmäßig durch Reduktion mit komplexen Hydriden wie LiAlH₄ oder NaBH₄.

Aromatische Vorstufen der Verbindungen der Formeln II und III können durch allgemein bekannte elektrophile Substitutionen umgewandelt werden. Eine bevorzugte Reaktion ist die Friedel-Crafts-Acylierung. Dabei wird die aromatische Verbindung in Dichlormethan mit einem aus Aluminiumchlorid und einem Acylchlorid gebildeten Komplex in ein Keton umgewandelt.

Nitrile der Formeln II und III werden durch Dehydratisierung von Amiden mit POCl₃ oder SOCl₂ in Dimethylformamid oder Toluol hergestellt. Die Amide -CONH₂ werden aus Carbonsäurehalogeniden oder Estern durch Umsetzung mit Ammoniak hergestellt. Die Carbonsäuren unter den Vorstufen werden vorzugsweise durch Oxidation von Aldehyden oder Ketonen heregestellt. Eine bevorzugte Methode ist die bekannte Haloformreaktion, bei der Verbindungen mit der Gruppe -COCH₃ in eine stark alkalische Lösung eines Alkalihypobromits eingebracht werden. Nach dem Ansäuern werden im allgemeinen gut kristallisierbare Carbonsäuren erhalten.

Die erfindungsgemäßen flüssigkristallinen Medien enthalten mindestens eine der Verbindungen der Formeln I, insbesondere II. Bevorzugt sind Mischungen, in denen 2 bis 10 solcher Verbindungen enthalten sind. Der Anteil, den diese Verbindungen in solchen Mischungen ausmachen können, beträgt zwischen 1 bis 100 Gew.%. Bevorzugt ist ein Anteil von 30 bis 98 Gew.%. Die Verbindungen der Formel II können enantiotrop oder monotrop flüssigkristallin sein. Neben Verbindungen der Formeln I und II können in erfindungsgemäßen Medien auch bekannte enantiotrop oder monotrop flüssigkristalline Verbindungen, die in reiner Form nematische, cholesterische, smektische, diskotische oder phasmidische Phasen bilden, enthalten sein. Sie werden vorzugsweise ausgewählt aus den Klassen der substituierten Phenylcyclohexane, Biphenyle, Bicyclohexyle, Phenylbicyclo[2.2.2]octane, Phenylcyclohexylcarboxylate, Phenylbenzoate, N-Benzylidenaniline, Azobenzole, Phenylpyridine, Phenyl- oder Cyclohexylpyrimidine, Tolane, Bicyclohexylphenyle, Cyclohexylbiphenyle und Dicyclohezylbiphenyle. Diese Mischungskomponenten können auch polymere flüssigkristalline Verbindungen sein.

Daneben können die erfindungsgemäßen flüssigkristallinen Medien weitere übliche Zusätze enthalten, wie z.B. dichroitische Farbstoffe für Farbanzeigen, Antioxidantien und nichtmesomorphe organische Verbindungen zur Herabsetzung der Viskosität. Daneben kommen optisch aktive Zusätze in Frage.

Die erfindungsgemäßen flüssigkristallinen Medien werden in an sich bekannter Weise hergestellt. Die Komponenten werden üblicherweise ineinander gelöst, vorteilhafterweise bei erhöhter Temperatur.

Die folgenden Beispiele erläutern die Erfindung näher, ohne sie zu begrenzen. Hierin bedeuten F.= Schmelzpunkt, K.= Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Beispiele 1 bis 7

1.) 19,0 g wasserfreies Aluminiumchlorid werden unter Feuchtigkeitsausschluß mit 75 ml Dichlormethan überschichtet. Bei Eiskühlung werden portionsweise 6,5 g Bernsteinsäureanhydrid unter Rühren hinzugefügt. Dazu wird eine Lösung von 20 g des bekannten 4-trans-(4-Pentylcyclohexyl)-biphenyls in 50 ml Dichlormethan getropft, wobei die Temperatur des Reaktionsgemisches 10° nicht überschreiten soll. Übliche Aufarbeitung (Umkristallisation aus Eisessig) ergibt 19,5 g 4-Oxo-4-[4'-trans-(4-pentylcyclohexyl)-biphenylyl-4]-buttersäure, F.215°, K.>320°.
   16,0 g dieser Säure werden in einem Gemisch aus 250 ml Tetrahydrofuran und 50 ml Ethanol gelöst. Die Hydrierung erfolgt nach Zugabe von 0,8 g Palladium-Katalysator (5% Pd auf Aktivkohle) bei Normaldruck und Raumtemperatur. Die Wasserstoffaufnahme ist nach 20 h beendet. Der Katalysator wird abfiltriert, das Lösungsmittel abdestilliert und der Rückstand aus Eisessig umkristallisiert. Ausbeute: 9,3 g 4-[4'-trans-(4-Pentylcyclohexyl)-biphenylyl-4]buttersäure, F.197°, K.210°.
   0,3 g Pentaerythrit werden in eine Schmelze von 4,0 g der Biphenylylbuttersäure bei 210° gebracht. Das Gemisch wird 4 h bei dieser Temperatur intensiv gerührt. Die abgekühlte Schmelze wird in Dichlormethan gelöst und säulenohromatographisch gereinigt (Kieselgel/Dichlormethan). Der aus der Hauptfraktion nach dem Abdestillieren des Lösungsmittels gewonnene Rückstand wird aus 2-Butanon umkristallisiert. Ausbeute: 1,5 g Tetrakis[4-(4'-trans-(4-pentylcyclohexyl)-biphenylyl-4)-butyryloxymethyl]methan, F.226°, K.283°.
   Auf die gleiche Weise werden, ausgehend von den entsprechenden Carbonsäuren und bei Mengenanpassung an die molaren Verhältnisse des Beispiels 1, hergestellt:
2.) Tetrakis[4-trans-(4-pentylcyclohexyl)-benzoyloxymethyl]methan, F.99°.
3.) Tetrakis[4'-propyl-biphenylyl-4-acetyloxymethyl]methan, F.168°, K.130°.
4.) Tetrakis[4-(4'-pentyl-biphenylyl-4)-butyryloxymethyl]methan, F.143°, K.120°.
5.) Tetrakis[4'pentyl-trans,trans-bicyclohexylyl-4-carbonyloxymethyl]methan, F.135°.
6.) Tetrakis[5-(4'-cyano-biphenylyl-4)-valeryloxymethyl]methan.
7.) Ausgehend von Phenyl-tris[hydroxymethyl]methan und 4-[4'-trans-(4-Pentylcyclohexyl)-biphenylyl-4]buttersäure wird hergestellt: Phenyl-tris[4-(4'-trans-(4-pentylcyclohexyl)-biphenylyl-4)-butyryloxymethyl]methan.

### Beispiele 8 bis 9

8.) 5,0 g des bekannten 4-(5-Heptyl-pyrimidyl-2)-phenols werden zusammen mit 1,35 g Pentaerythrityltetrabromid in 70 ml Dimethylsulfoxid gelöst. Nach Zugabe von 15 g feingepulvertem Kaliumcarbonat wird 16 h bei 80° gerührt. Übliche Aufarbeitung ergibt 2,1 g Tetrakis[4-(5-heptyl-pyrimidyl-2-)-phenyloxymethyl]methan, F.179°.
9.) Auf die gleiche Weise wird, ausgehend von Pentaerythrityltetrabromid und 4-(4-Decyloxybenzoyloxy)-phenol, Tetrakis[4-(4-decyloxybenzoyloxy)-phenyloxymethyl]-methan hergestellt.

### Beispiel 10 (Anwendungsbeispiel)

Ein flüssigkristallines Medium, bestehend aus
20,0% Tetrakis[4-(4'-pentyl-biphenylyl-4)-butyryloxymethyl]methan
27,5% Tetrakis[4'-propyl-biphenylyl-4-acetoxy-methyl]methan
49,0% 4'-trans-(4-Pentylcyclohexyl)-biphenylyl-4-carbonitril
3,5% 4,4'-Bis[trans-4-propylcyclohexyl]-2-fluor-biphenyl
hat einen Übergang von smektisch A zu nematisch bei 143° und einen Übergang von nematisch zu flüssig bei 151°. Die smektische Phase ist auf Raumtemperatur unterkühlbar. Aufgrund seiner positiven Dielektrizitätsanisotropie eignet es sich vorteilhaft zur Anwendung in einem löschbaren optischen Speicherelement. Beim Abkühlen einer dünnen Schicht des Mediums von der nematischen in die smektische Phase in einem ausreichend starken elektrischen Feld bleibt diese transparent. Beim Abkühlen ohne elektrisches Feld ist sie stark lichtstreuend.

## Patentansprüche

1. Methanderivate der allgemeinen Formel
C[-Sp-M-R]₄ (I)
wobei die Reste -Sp-M-R gleich oder verschieden sein können, und
Sp
jeweils unabhängig voneinander einen unsubstituierten oder einen einfach mit Halogen, -CN oder -CF₃ substituierten Alkylen- oder Alkenylenrest mit 1 bis 12 Kohlenstoffatomen, worin auch eine oder zwei nichtbenachbarte CH₂-Gruppen durch -O-, -CO-, -COO-, -OOC-, -CONH- oder -OCOO- ersetzt sein können, oder eine Einfachbindung;
M
eine mesogene Gruppe der allgemeinen Formel -A-(Z₁-A₁)ₘ-(Z₂-A₂)ₙ- bedeutet, in der
Z₁, Z₂
jeweils unabhängig voneinander -CH₂CH₂-, -COO-, -OOC-, -OCH₂-, -CH₂O-, -C≡C-, -N=N-oder eine Einfachbindung;
A, A₁, A₂
jeweils unabhängig voneinander einen unsubstituierten oder einen bis vierfach mit Fluor oder Chlor substituierten 1,4-Phenylenrest, worin auch eine oder zwei CH-Gruppen durch N ersetzt sein können, einen insbesondere trans-Konfiguration aufweisenden 1,4-Cyclohexylenrest, worin auch eine oder zwei nicht benachbarte CH₂-Gruppen durch -O- ersetzt sein können, einen Piperidin-1,4-diylrest oder einen 1,4-Bicyclo[2.2.2]octylenrest; sowie
n und m
jeweils unabhängig voneinander 0 oder 1, mit der Maßgabe, daß in einem der vier Substituenten des Methans m oder n mindestens einmal 1 ist,
bedeuten; und
R
Wasserstoff, Halogen, -CN, -CF₃, -OCF₃, -NO₂, -N(CH₃)₂, einen unsubstituierten, einen einfach oder mehrfach durch -CN, -NH₂, -CF₃ oder Halogen substituierten, gerad- oder verzweigtkettigen Alkylrest oder Alkenylrest mit jeweils 1 bis 12 C-Atomen, in denen eine oder mehrere nichtbenachbarte CH₂-Gruppen durch die Reste -O-, -CO-, -COO-, -OOC- oder -OCOO- ersetzt sein können,
bedeuten.

2. Methanderivate nach Anspruch 1, dadurch gekennzeichnet, daß Sp einen Rest der Formel -CH₂COO(CH₂)ᵣ- oder -CH₂O(CH₂)ₛ, wobei r bzw. s die Zahlen 0 bis 10 sein können, bedeutet.

3. Methanderivate nach einem der vorangehenden Ansprüche 1 und 2, dadurch gekennzeichnet, daß A, A₁ und/oder A₂ einen 1,4-Phenylenrest, einen 1,4-Cyclohexylenrest oder einen Pyrimidin-2,5-diylrest bedeuten.

4. Methanderivate nach einem der vorangehenden Ansprüche 1 bis 3, dadurch gekennzeichnet, daß Z₁ und Z₂ jeweils die Einfachbindung oder einen der Reste -CH₂CH₂-, -COO- und -CH₂O- bedeuten.

5. Methanderivate nach einem der vorangehenden Ansprüche 1 bis 4 dadurch gekennzeichnet, daß R eine Alkylgruppe mit 1 bis 12 Kohlenstoffatomen, -CN oder -F bedeuten.

6. Methanderivate nach einem der Ansprüche 1 bis 5, dadurch gekennzeichnet, daß drei Substituenten
-[Sp-A-(Z₁-A₁)ₘ-(Z₂-A₂)ₙ-R] gleich sind.

7. Methanderivate nach Anspruch 1, dadurch gekennzeichnet, daß alle vier Substituenten
-[Sp-A-(Z₁-A₁)ₘ-(Z₂-A₂)ₙ-R] gleich sind.

8. Methanderivate nach Anspruch 7, dadurch gekennzeichnet, daß Z₁ eine Einfachbindung, m = 1, und n = Null sind.

9. Methanderivate nach Anspruch 7, dadurch gekennzeichnet, daß m = 1, und n = Null sind.

10. Methanderivate nach Anspruch 7, dadurch gekennzeichnet, daß m und n = 1, und Z₁ und Z₂ Einfachbindungen sind.

11. Methanderivate nach Anspruch 8, dadurch gekennzeichnet, daß A und A₁ jeweils ein ggf. mono- bis tetrachlorierter oder -fluorierter 1,4-Phenylenrest sind.

12. Methanderivate nach Anspruch 8, dadurch gekennzeichnet, daß A ein ggf. mono- bis tetrachlorierter oder -fluorierter 1,4-Phenylen-, und A₁ ein ggf. mono- bis tetrachlorierter oder -fluorierter 1,4-Cyclohexylenrest sind.

13. Methanderivate nach Anspruch 8, dadurch gekennzeichnet, daß A und A₁ jeweils ein ggf. mono- bis tetrachlorierter oder -fluorierter 1,4-Cyclohexylenrest sind.

14. Methanderivate nach Anspruch 9, dadurch gekennzeichnet, daß Z₁ ein Ethylenrest, A ein ggf. mono- bis tetrachlorierter oder -fluorierter 1,4-Phenylen-, und A₁ ein ggf. mono- bis tetrachlorierter oder -fluorierter 1,4-Cyclohexylenrest sind.

15. Methanderivate nach Anspruch 9, dadurch gekennzeichnet, daß A und A₁ jeweils ein ggf. mono- bis tetrachlorierter oder -fluorierter 1,4-Phenylenrest, und Z₁ ein Carbonyloxyrest sind.

16. Methanderivate nach Anspruch 10, dadurch gekennzeichnet, daß A und A₁ jeweils ein ggf. mono- bis tetrachlorierter oder -fluorierter 1,4-Phenylenrest, und A₂ ein ggf. mono- bis tetrachlorierter oder -fluorierter 1,4-Cyclohexylenrest sind.

17. Methanderivate nach Anspruch 10, dadurch gekennzeichnet, daß A ein ggf. mono- bis tetrachlorierter oder -fluorierter 1,4-Phenylenrest, und A₁ und A₂ jeweils ein ggf. mono- bis tetrachlorierter oder -fluorierter 1,4-Cyclohexylenrest sind.

18. Methanderivate nach Anspruch 10, dadurch gekennzeichnet, daß A ein ggf. mono- bis tetrachlorierter oder -fluorierter 1,4-Phenylenrest, und A₁ und A₂ jeweils ein ggf. mono- bis tetrachlorierter oder -fluorierter 1,4-Cyclohexylenrest sind.

19. Methanderivate nach einem der Ansprüche 12 bis 14 und 16 bis 17, dadurch gekennzeichnet, daß der 1,4-Cyclohexylenrest trans-Konfiguration aufweist.

20. Methanderivate nach einem der Ansprüche 7 bis 19, dadurch gekennzeichnet, daß Sp die Einfachbindung oder einen der Reste -CH₂O-, -CH₂OOC-, -CH₂O(CH₂)ᵣ-,
-CH₂OOC(CH₂)ₛ-, wobei r und s jeweils 0 bis 10, insbesondere 1 bis 6 sind, bedeuten.

21. Methanderivate nach einem der Ansprüche 7 bis 20, dadurch gekennzeichnet, daß R einen gerad- oder verzweigtkettigen Alkylrest, einen Alkoxy- oder Alkoxymethylrest bedeuten.

22. Methanderivate nach Anspruch 21, dadurch gekennzeichnet, daß R ein unverzweigter Alkylrest mit 1 bis 7 Kohlenstoffatomen ist.

23. Tetrakis[4-(4'-trans-(4-pentylcyclohexyl)-biphenylyl-4)-butyryloxymethyl]methan.

24. Tetrakis[4-trans-(4-pentylcyclohexyl)-benzoyloxymethyl]methan.

25. Tetrakis[4'-propyl-biphenylyl-4-acetyloxymethyl]methan.

26. Tetrakis[4-(4'-pentyl-biphenylyl-4)-butyryloxymethyl]methan.

27. Tetrakis[4'-pentyl-trans,trans-bicyclohexylyl-4-carbonyloxymethyl]methan.

28. Tetrakis[5-(4'-cyano-biphenylyl-4)-valeryloxymethyl)methan.

29. Phenyl-tris[4-(4'-trans-(4-pentylcyclohexyl)-biphenylyl-4)-butyryloxymethyl]methan.

30. Tetrakis[4-(5-heptyl-pyrimidyl-2)-phenyloxymethyl]methan.

31. Tetrakis[4-(4-decyloxybenzoyloxy)-phenyloxymethyl]methan.

32. Flüssigkristallines Medium, enthaltend eine oder mehrere Verbindungen nach einem der vorangehenden Ansprüche 1 bis 31.

33. Flüssigkristallines Medium nach Anspruch 32, gekennzeichnet durch einem Gehalt an einer oder mehreren Verbindungen gemäß Ansprüchen 23 bis 31.

34. Verwendung des flüssigkristallinen Mediums nach einem der Ansprüche 32 bis 33 in einem elektrooptischen Anzeigeelement, einer Temperaturanzeigevorrichtung auf Basis selektiver Lichtreflexion, einem optischen Speicherelement oder einem nichtlinear-optischen Schaltelement.

## Claims

1. Methane derivative of the general formula
C[-Sp-M-R]₄ (I)
in which the radicals -Sp-M-R may be identical or different and where
Sp is, in each case independently of the other cases, an alkylene or alkenylene radical having 1 to 12 carbon atoms which is unsubstituted or monosubstituted by halogen, -CN or -CF₃ and in which, in addition, one or two non-adjacent CH₂ groups may be replaced by -O-, -CO-, -COO-, -OOC-, -CONH- or -OCOO-, or is a single bond;
M is a mesogenic group of the general formula
-A-(Z₁-A₁)ₘ-(Z₂-A₂)ₙ- in which
Z₁ and Z₂ are each, independently of one another, -CH₂CH₂-, -COO-, -OOC-, -OCH₂-, CH₂O-, -C≡C-, -N=N- or a single bond;
A, A₁ and A₂ are each, independently of one another, a 1,4-phenylene radical which is unsubstituted or monosubstituted to tetrasubstituted by fluorine or chlorine and in which, in addition, one or two CH groups may be replaced by N, or are a 1,4-cyclohexylene radical which has, in particular, a trans-configuration and in which, in addition, one or two non-adjacent CH₂ groups may be replaced by -O-, or are a piperidine-1,4-diyl radical or a 1,4-bicyclo[2.2.2]octylene radical; and
n and m are each, independently of one another, 0 or 1, with the proviso that m or n is 1 at least once in one of the four substituents of the methane; and
R is hydrogen, halogen, -CN, -CF₃, -OCF₃, -NO₂, -N(CH₃)₂, a straight-chain or branched alkyl or alkenyl radical having 1 to 12 carbon atoms which is unsubstituted or monosubstituted or polysubstituted by -CN, -NH₂, -CF₃ or halogen and in which one or more non-adjacent CH₂ groups may be replaced by the radicals -O-, -CO-, -COO-, -OOC- or -OCOO-.

2. Methane derivative according to Claim 1, characterized in that Sp is a radical of the formula -CH₂COO(CH₂)ᵣ- or -CH₂O(CH₂)ₛ, where r and s may be the numbers 0 to 10.

3. Methane derivative according to one of the preceding Claims 1 and 2, characterized in that A, A₁ and/or A₂ are a 1,4-phenylene radical, a 1,4-cyclohexylene radical or a pyrimidine-2,5-diyl radical.

4. Methane derivative according to one of the preceding Claims 1 to 3, characterized in that Z₁ and Z₂ are each a single bond or one of the radicals -CH₂CH₂-, -COO- and -CH₂O-.

5. Methane derivative according to one of the preceding Claims 1 to 4, characterized in that R is an alkyl group having 1 to 12 carbon atoms, -CN or -F.

6. Methane derivative according to one of Claims 1 to 5, characterized in that three substituents -[Sp-A-(Z₁-A₁)ₘ-(Z₂-A₂)ₙ-R] are identical.

7. Methane derivative according to Claim 1, characterized in that all four substituents -[Sp-A-(Z₁-A₁)ₘ-(Z₂-A₂)ₙ-R] are identical.

8. Methane derivative according to Claim 7, characterized in that Z₁ is a single bond, m = 1 and n = zero.

9. Methane derivative according to Claim 7, characterized in that m = 1 and n = zero.

10. Methane derivative according to Claim 7, characterized in that m and n = 1, and Z₁ and Z₂ are single bonds.

11. Methane derivative according to Claim 8, characterized in that A and A₁ are each an optionally mono- to tetrachlorinated or -fluorinated 1,4-phenylene radical.

12. Methane derivative according to Claim 8, characterized in that A is an optionally mono- to tetrachlorinated or -fluorinated 1,4-phenylene radical, and A₁ is an optionally mono- to tetrachlorinated or -fluorinated 1,4-cyclohexylene radical.

13. Methane derivative according to Claim 8, characterized in that A and A₁ are each an optionally mono- to tetrachlorinated or -fluorinated 1,4-cyclohexylene radical.

14. Methane derivative according to Claim 9, characterized in that Z₁ is an ethylene radical, A is an optionally mono- to tetrachlorinated or -fluorinated 1,4-phenylene radical, and A₁ is an optionally mono- to tetrachlorinated or -fluorinated 1,4-cyclohexylene radical.

15. Methane derivative according to Claim 9, characterized in that A and A₁ are each an optionally mono- to tetrachlorinated or -fluorinated 1,4-phenylene radical, and Z₁ is a carbonyloxy radical.

16. Methane derivative according to Claim 10, characterized in that A and A₁ are each an optionally mono- to tetrachlorinated or -fluorinated 1,4-phenylene radical, and A₂ is an optionally mono- to tetrachlorinated or -fluorinated 1,4-cyclohexylene radical.

17. Methane derivative according to Claim 10, characterized in that A is an optionally mono- to tetrachlorinated or -fluorinated 1,4-phenylene radical, and A₁ and A₂ are each an optionally mono- to tetrachlorinated or -fluorinated 1,4-cyclohexylene radical.

18. Methane derivative according to Claim 10, characterized in that A is an optionally mono- to tetrachlorinated or -fluorinated 1,4-phenylene radical, and A₁ and A₂ are each an optionally mono- to tetrachlorinated or -fluorinated 1,4-cyclohexylene radical.

19. Methane derivative according to one of Claims 12 to 14 and 16 to 17, characterized in that the 1,4-cyclohexylene radical has the trans-configuration.

20. Methane derivative according to one of Claims 7 to 19, characterized in that Sp is a single bond or one of the radicals -CH₂O-, -CH₂OOC-, -CH₂O(CH₂)ᵣ- or -CH₂OOC(CH₂)ₛ-, where r and s are each 0 to 10, in particular 1 to 6.

21. Methane derivative according to one of Claims 7 to 20, characterized in that R is a straight-chain or branched alkyl radical, an alkoxy radical or an alkoxy methyl radical.

22. Methane derivative according to Claim 21, characterized in that R is an unbranched alkyl radical having 1 to 7 carbon atoms.

23. Tetrakis[4-(4'-trans-(4-pentylcyclohexyl)-biphenyl-4-yl)butyryloxymethyl]methane.

24. Tetrakis[4-trans-(4-pentylcyclohexyl)benzoyloxymethyl]methane.

25. Tetrakis[4'-propylbiphenyl-4-yl-acetyloxymethyl]methane.

26. Tetrakis[4-(4'-pentyl-biphenyl-4-yl)butyryloxymethyl]methane.

27. Tetrakis[4'-pentyl-trans,trans-bicyclohexyl-4-yl-carbonyloxymethyl]methane.

28. Tetrakis[5-(4'-cyano-biphenyl-4-yl)valeryloxymethyl]methane.

29. Phenyltris[4-(4'-trans-(4-pentylcyclohexyl)-biphenyl-4-yl)butyryloxymethyl]methane.

30. Tetrakis[4-(5-heptyl-2-pyrimidyl)phenyloxymethyl]methane.

31. Tetrakis[4-(4-decyloxybenzoyloxy)phenyloxymethyl]methane.

32. Liquid-crystalline medium containing one or more compounds according to one of the preceding Claims 1 to 31.

33. Liquid-crystalline medium according to Claim 32, characterized by a content of one or more compounds according to Claims 23 to 31.

34. The use of a liquid-crystalline medium according to one of Claims 32 and 33 in an electro-optical display element, a temperature-display device based on selective light reflection, an optical memory element or a non-linear optical switching element.

## Revendications

1. Dérivés du méthane répondant à la formule générale :
C[-Sp-M-R]₄ (I)
dans laquelle les radicaux -Sp-M-R peuvent être identiques ou différents, et
les symboles Sp désignent chacun indépendamment l'un de l'autre un radical alkylène ou alcénylène en C₁-C₁₂ non substitué ou substitué une fois par un atome d'halogène, un groupe -CN ou -CF₃, dans lequel un ou deux groupes CH₂ non voisins peuvent aussi être remplacés par -O-, -CO-, -COO-, -OOC-, -CONH- ou -OCOO-, ou une simple liaison,
M désigne un groupe mésogène répondant à la formule générale :
-A-(Z₁-A₁)ₘ-(Z₂-A₂)ₙ-
dans laquelle
Z₁, Z₂ désignent chacun indépendamment l'un de l'autre les groupes -CH₂CH₂-, -COO-, -OOC-, -OCH₂-, CH₂O-, -C≡C-, -N=N- ou une simple liaison ;
A, A₁, A₂ désignent chacun indépendamment l'un de l'autre un radical 1,4-phénylène non substitué ou substitué une à quatre fois par des atomes de fluor ou de chlore, dans lequel un ou deux groupes CH peuvent aussi être remplacés par N, un radical 1,4-cyclohexylène présentant en particulier une configuration trans, dans lequel un ou deux groupes CH₂ non voisins peuvent également être remplacés par -O-, un radical pipéridine-1,4-diyle ou un radical 1,4-bicyclo[2.2.2]octylène ; et
n et m désignent chacun indépendamment l'un de l'autre 0 ou 1, sous réserve que dans un des quatre substituants du méthane, m ou n est au moins une fois égal à 1 ; et
R désigne un atome d'hydrogène, un atome d'halogène, un groupe -CN, CF₃, -OCF₃, -NO₂, -N(CH₃)₂, un radical alkyle ou alcényle en C₁-C₁₂ linéaire ou ramifié, non substitué ou substitué une ou plusieurs fois par un groupe -CN, -NH₂, -CF₃ ou un atome d'halogène, dans lequel un ou plusieurs groupes CH₂ non voisins peuvent être remplacés par les radicaux -O-, -CO-, -COO-, -OOC- ou -OCOO-.

2. Dérivés du méthane selon la revendication 1, caractérisés en ce que Sp désigne un radical répondant aux formules -CH₂COO(CH₂)ᵣ- ou -CH₂O(CH₂)ₛ, dans lesquelles r ou s peuvent être les nombres 0 à 10.

3. Dérivés du méthane selon l'une des revendications 1 et 2 précédentes, caractérisés en ce que A, A₁ et/ou A₂ désignent un radical 1,4-phénylène, un radical 1,4-cyclohexylène ou un radical pyrimidine-2,5-diyle.

4. Dérivés du méthane selon l'une des revendications 1 à 3, caractérisés en ce que Z₁ et Z₂ désignent chacun une liaison simple ou un des radicaux -CH₂CH₂-, -COO- et -CH₂O-.

5. Dérivés du méthane selon l'une des revendications 1 à 4 précédentes, caractérisés en ce que R désigne un groupe alkyle en C₁-C₁₂, -CN ou -F.

6. Dérivés du méthane selon l'une des revendications 1 à 5, caractérisés en ce que trois substituants -[Sp-A-(Z₁-A₁)ₘ-(Z₂-A₂)ₙ-R] sont identiques.

7. Dérivés du méthane selon la revendication 1, caractérisés en ce que les quatre substituants -[SP-A-(Z₁-A₁)ₘ-(Z₂-A₂)ₙ-R] sont tous identiques.

8. Dérivés du méthane selon la revendication 7, caractérisés en ce que Z₁ est une liaison simple, m est 1 et n est 0.

9. Dérivés du méthane selon la revendication 7, caractérisés en ce que m est 1 et n est 0.

10. Dérivés du méthane selon la revendication 7, caractérisés en ce que m et n sont 1 et Z₁ et Z₂ des liaisons simples.

11. Dérivés du méthane selon la revendication 8, caractérisés en ce que A et A₁ sont chacun un radical 1,4-phénylène le cas échéant mono- à tétrachloré ou -fluoré.

12. Dérivés du méthane selon la revendication 8, caractérisés en ce que A est un groupe 1,4-phénylène le cas échéant mono- à tétrachloré ou -fluoré et A₁ un radical 1,4-cyclohexylène le cas échéant mono- à tétrachloré ou -fluoré.

13. Dérivés du méthane selon la revendication 8, caractérisés en ce que A et A₁ sont chacun un radical 1,4-cyclohexylène le cas échéant mono- à tétrachloré ou -fluoré.

14. Dérivés du méthane selon la revendication 9, caractérisés en ce que Z₁ est un radical éthylène, A un radical 1,4-phénylène le cas échéant mono- à tétrachloré ou -fluoré, et A₁ est un radical 1,4-cyclohexylène le cas échéant mono- à bis-tétrachloré ou -fluoré.

15. Dérivés du méthane selon la revendication 9, caractérisés en ce que A et A₁ sont chacun un radical 1,4-phénylène mono- à tétrachloré ou -fluoré, et Z₁ est un radical carbonyloxy.

16. Dérivés du méthane selon la revendication 10, caractérisés en ce que A et A₁ sont chacun un radical 1,4-phénylène le cas échéant mono- à tétrachloré ou -fluoré, et A₂ est un radical 1,4-cyclohexylène le cas échéant mono- à bis-tétrachloré ou -fluoré.

17. Dérivés du méthane selon la revendication 10, caractérisés en ce que A est un radical 1,4-phénylène le cas échéant mono- à tétrachloré ou -fluoré, et A₁ et A₂ sont chacun un radical 1,4-cyclohexylène le cas échéant mono- à tétrachloré ou -fluoré.

18. Dérivés du méthane selon la revendication 10, caractérisés en ce que A est un radical 1,4-phénylène le cas échéant mono- à tétrachloré ou -fluoré, et A₁ et A₂ sont chacun un radical 1,4-cyclohexylène le cas échéant mono- à tétrachloré ou -fluoré.

19. Dérivés du méthane selon l'une des revendications 12 à 14 et 16 à 17, caractérisés en ce que le radical 1,4-cyclohexylène présente la configuration trans.

20. Dérivés du méthane selon l'une des revendications 7 à 19, caractérisés en ce que Sp désigne une liaison simple ou un des radicaux -CH₂O-, -CH₂OOC-, -CH₂O(CH₂)ᵣ-, -CH₂OOC(CH₂)ₛ-, dans lesquels r et s sont chacun un nombre de 0 à 10, en particulier de 1 à 6.

21. Dérivés du méthane selon l'une des revendications 7 à 20, caractérisés en ce que R est un radical alkyle linéaire ou ramifié, un radical alcoxy ou alcoxyméthyle.

22. Dérivés du méthane selon la revendication 21, caractérisés en ce que R est un radical alkyle non ramifié en C₁-C₇.

23. Tétrakis[4-(4'-trans-(4-pentylcyclohexyl)-biphénylyl-4)-butyryloxyméthyl]-méthane.

24. Tétrakis-[4-trans-(4-pentylcyclohexyl)-benzoyloxyméthyl]-méthane.

25. Tétrakis-(4'-propyl-biphénylyl-4-acétyloxyméthyl]-méthane.

26. Tétrakis-(4-(4'-pentyl-biphénylyl-4)-butyryloxyméthyl]-méthane.

27. Tétrakis-[4'-pentyl-trans-trans-bicyclohexylyl-4-carbonyloxyméthyl]-méthane.

28. Tétrakis-[5-(4'-cyano-biphénylyl-4)-valéryloxyméthyl]-méthane.

29. Phényl-tris[4-(4'-trans-(4-pentylcyclohexyl)-biphénylyl-4)-butyryloxyméthyl]méthane.

30. Tétrakis-[4-(5-heptyl-pyrimidyl-2)-phényloxyméthyl]-méthane.

31. Tétrakis-[4-(4-décyloxybenzoyloxy)-phényloxyméthyl]-méthane.

32. Milieu cristallin liquide contenant un ou plusieurs composés selon l'une des revendications 1 à 31 qui précèdent.

33. Milieu criscallin liquide selon la revendication 32, caractérisé qu'il contient un ou plusieurs composés selon les revendications 23 à 31.

34. Utilisation du milieu cristallin liquide selon l'une des revendications 32 à 33 dans un élément d'affichage électrooptique, dans un dispositif d'affichage des températures basé sur une réflexion sélective de la lumière, dans un élément de mémoire optique ou dans un élément de commutation optique non linéaire.
